# EUROPEAN PATENT SPECIFICATION

(11) **EP 2 655 655 B1**
(45) Date of publication and mention of the grant of the patent: **09.03.2016**
(21) Application number: 11804760.4
(22) Date of filing: 20.12.2011
(51) Int. Cl.: C12Q 1/68

(54) **DETECTION OF QUANTITATIVE GENETIC DIFFERENCES**
NACHWEIS VON QUANTITATIVEN GENETISCHEN UNTERSCHIEDEN
DÉTECTION DE DIFFÉRENCES GÉNÉTIQUES QUANTITATIVES

(30) Priority: 20.12.2010 GB 201021499
(43) Date of publication of application: 30.10.2013
(73) Proprietor: Premaitha Limited, Manchester M15 6SZ (GB)
(72) Inventor: OLD, Robert, London W1S 4BS (GB); CREA, Francesco, London W1S 4BS (GB); ROBERTS, Charles, London W1S 4BS (GB)
(74) Representative: Gibson, Mark
(86) International application number: PCT/GB2011/052525
(87) International publication number: WO 2012/085554

(56) References cited:
- WO-A1-2007/048469
- WO-A1-2009/127408
- LEE H-H ET AL: "Rapid detection of trisomy 21 by homologous gene quantitative PCR (HGQ-PCR)", HUMAN GENETICS, SPRINGER, BERLIN, DE, vol. 99, 1 January 1997 (1997-01-01), pages 364-367, XP003015784, ISSN: 1432-1203, DOI: 10.1007/S004390050373
- LICHTER P ET AL: "Rapid detection of human chromosome 21 abberations by in situ hybridization", PROCEEDINGS OF THE NATIONAL ACADEMY OF SCIENCES OF USA, NATIONAL ACADEMY OF SCIENCE, WASHINGTON, DC; US, vol. 85, no. 24, 1 December 1988 (1988-12-01), pages 9664-9668, XP002115018, ISSN: 0027-8424, DOI: 10.1073/PNAS.85.24.9664
- SINGER ALON ET AL: "Nanopore based sequence specific detection of duplex DNA for genomic profiling.", NANO LETTERS 10 FEB 2010 LNKD- PUBMED:20088590, vol. 10, no. 2, 10 February 2010 (2010-02-10), pages 738-742, XP002668690, ISSN: 1530-6992 cited in the application

## Description

### FIELD OF THE INVENTION

The present invention relates to uses and methods for the detection of a quantitative difference between first and second target sequence regions present in a nucleic acid sample, in particular but not exclusively a DNA sample, and a kit for carrying out the uses and methods of the invention.

### BACKGROUND OF THE INVENTION

The detection of small differences in nucleic acid content can be very important in detecting and diagnosing a disease or a predisposition to a disease. The differences may be differences in gene dosage or chromosome number. However the detection of small differences is very difficult.

Gene dosage differences in somatic cells are exemplified by chromosomal abnormalities resulting in gain or loss of genetic material often observed in malignant tumours. Among these abnormalities, oncogene amplification (increase of gene copy number) is regarded as one of the most important mechanisms of oncogene activation in carcinogenesis. Successful detection of amplified genes can be useful diagnostically and appears to be especially important for predicting chemotherapy responsiveness in a number of malignancies. Correct selection of treatment strategies in individual cases can significantly improve life expectancy in patients with advanced tumours. The role of HER2/neu (c-erbB-2) gene amplification in determining breast cancer sensitivity to chemotherapy can serve as a good example.

However, in many cases it is difficult to reliably detect gene amplification in tumours since tumour tissue or cell samples obtained from patients are usually characterised by a strong presence of non-malignant cellular elements representing connective tissue, blood and lymphoid cells, inflammatory cells etc. For this reason, analysis of gene amplification in this material requires high sensitivity, to allow detection of the presence of extra copies of target gene(s) in mixed samples of malignant and normal cells provided by surgical removal of tumours, biopsies, body fluid sampling etc.

Hereditary numerical chromosome abnormalities, that is an increase or decrease in the number of a particular chromosome, are known to cause a number of syndromes, which may lead to physical or mental disability in life (syndromes of Down, Klinefelter, Edward, Patau, XXX, XXY etc.). Therefore it is desirable to detect if an unborn child, in particular a foetus, has such an abnormality. Knowing the likelihood of disease can provide useful information to the parents who may wish to terminate the pregnancy or to prepare for caring for a disabled child.

Although cell free fetal DNA (cff DNA) is routinely used to determine fetal sex, for rhesus D status analysis, and for the diagnosis of rare genetic disorders e.g. β-thalassemia, no one has been able to apply this technology for the diagnosis of more common genetic conditions, due to the vast background of maternal cell free DNA. Some parties are attempting to either use SNPs in conjunction with fetal-specific cff RNA or fetal specific epigenetic markers e.g methylation, to select for fetal-specific cff DNA. However, SNPs can only be used to score foetuses that are heterozygous at the target SNP, so limiting the percentage of the population that can be tested. In addition, epigenetic markers are influenced by the exposure of the mother to environmental factors, which could in principle influence the epigenetic status of the foetus.

Conventional tests for chromosome abnormalities of foetuses are invasive because they require a medical professional to obtain cell material directly from the foetus or amniotic fluid surrounding the foetus. Such invasive procedures can lead to complications or even termination of the pregnancy.

It is desirable to provide a test for fetal numerical chromosome abnormality and/or gene dosage differences which is non-invasive, for example, by testing a sample of maternal blood, which will have fetal nucleic acid present in it. Fetal DNA can be found in maternal blood plasma early in pregnancy and its transplacental transition appears to be increased in cases of fetal chromosome abnormalities. However, it can be difficult to detect an imbalance in chromosome and/or gene copy numbers using maternal blood because the increased or decreased copy number of the target gene or chromosome observed in abnormal fetal DNA is likely to be masked by the presence of normal maternal DNA in excess. Conventional quantitative real time PCR is not sensitive enough to detect the small difference in gene or chromosome copy number, because only a small fraction of the template DNA is from the fetus.

The most common numerical chromosome abnormality known as Down's syndrome is caused by an imbalance in chromosome copy number where a patient has an additional copy of chromosome 21 (trisomy 21). A pure fetal DNA sample of a Down's syndrome afflicted subject would have 3:2 ratio of chromosome 21 to another chromosome, such as chromosome 1. Whereas in a maternal blood sample comprising a mixture of maternal and trisomic fetal DNA, the ratio of chromosome 21 to another chromosome would be much less at (2+x):2 (where x is a fraction of 1 corresponding to the share of trisomic fetal DNA in the sample), because the relative large amount of maternal DNA dominates the total DNA content of the maternal blood sample. For example, if fetal DNA provided 15% of the DNA in a maternal blood sample from a mother with a Down's syndrome-affected fetus, this would give the ratio of chromosome 21 to any other chromosome of 2.15:2. This ratio is far below the detection limit of conventional real time PCR. Thus, improved methods of DNA detection are required to observe these differences.

WO2009/127408 describes a method for the quantitative determination of the number of at least one predetermined sequence in a biological sample, in particular to a method for the determination of the absolute number of copies of alleles per cell. Singer et al (2010) Nano Lett. 10, 738-742 describes a nanopore based detection method for detecting specific DNA sequences by hybridizing double-stranded DNA with peptide nucleic acid (PNA) probes.

### SUMMARY OF THE INVENTION

According to a first aspect of the invention, there is provided a method for the detection of a quantitative difference between the amount of a first target region of nucleic acid and a second target region of nucleic acid in a sample, such that the first target region is a gene or chromosome the relative copy number of which is to be studied, and the second target region is a different gene or chromosome present in normal copy number, wherein said method comprises the steps of:
(a) providing the sample comprising the nucleic acid;
(b) hybridisation of one or more first probes to the first target region;
(c) hybridisation of one or more second probes to the second target region, such that the number and/or positioning of second probes on the second target region differs from the number and/or positioning of first probes on the first target region;
(d) detection of the number and/or position of the first probes identified on nucleic acid molecules within the sample as a specific signature assignable to the presence of the first target region;
(e) detection of the number and/or position of the second probes identified on nucleic acid molecules within the sample as a specific signature assignable to the presence of the second target region;

wherein a difference between the number of specific signatures obtained in step (d) and the number of specific signatures obtained in step (e) is indicative of a quantitative difference between the amount of the first and second target regions of nucleic acid in the sample; and
wherein the detection steps (d) and (e) comprise a nanopore based detection method.

According to a second aspect of the invention, there is provided a method for detection of an abnormality in a gene or chromosome copy number in a sample, comprising the steps of:
(a) providing the sample comprising the nucleic acid;
(b) hybridisation of one or more first probes to the first target region;
(c) hybridisation of one or more second probes to the second target region, such that the first target region is a gene or chromosome the relative copy number of which is to be studied, and the second target region is a different gene or chromosome present in normal copy number, and such that the number and/or positioning of second probes on the second target region differs from the number and/or positioning of first probes on the first target region;
(d) detection of the number and/or position of the first probes identified on specific nucleic acid molecules within the sample as a specific signature assignable to the presence of the first target region;
(e) detection of the number and/or position of the second probes identified on specific nucleic acid molecules within the sample as a specific signature assignable to the presence of the second target region;

wherein a difference between the number of specific signatures obtained in step (d) and the number of specific signatures obtained in step (e) is indicative of a quantitative difference between the amount of the first and second target regions of nucleic acid in the sample, and wherein the detection of a quantitative difference is indicative of an abnormality in a gene or chromosome copy number; and
wherein the detection steps (d) and (e) comprise a nanopore based detection method.

According to a further aspect of the invention, there is provided a use of a method as herein defined to determine if an individual has an increase or decrease in gene or chromosome copy number.

### BRIEF DESCRIPTION OF THE FIGURES

**Figure 1** describes a schematic representation of the first aspect of the invention.
**Figures 2-5** describe a pictorial representation of one embodiment of the method of the invention.

### DETAILED DESCRIPTION OF THE INVENTION

Herein disclosed is the use of a single molecule analysing nanopore complex comprising at least one nanopore in the detection of a quantitative difference between the amount of a first target region of nucleic acid and a second target region of nucleic acid in a sample.

The use of nanopore complexes in DNA sequencing has been demonstrated by threading an individual single stranded DNA (ssDNA) molecule through a staphylococcal α-haemolysin (αHL) protein pore under an applied potential while recording modulations of the ionic current passing through the pore (Clarke, J. et al (2009) Nature Nanotechnology 4, 265-270). Each base is registered in sequence by a characteristic decrease in current amplitude. Therefore, the nanopore sequencing method has the ability to perform single counting of individual molecules within nucleic acid. However, the use of nanopore technology has been restricted solely to the identification of specific nucleotide residues primarily for the purpose of sequencing and has not been applied to the detection of quantitative differences between given nucleic acid samples. The invention is therefore directed to the use of nanopore technology to perform single molecule counting upon given nucleic acid samples to detect quantitative differences between the samples. The invention therefore finds a particular and surprising utility in prenatal diagnosis of a fetal chromosome imbalance or abnormality.

References herein to the term "nanopore complex" refer to a structure comprising (a) a first and a second compartment separated by a physical barrier, which barrier has at least one pore with a diameter, for example, of from about 1 to 10 nm, and (b) a means for applying an electric field across the barrier so that a charged molecule, such as DNA or RNA optionally hybridized with PNA or bis-PNA, can pass from the first compartment through the pore to the second compartment. The nanopore ideally further comprises a means for measuring the electronic signature of a molecule passing through its barrier. The nanopore barrier may be synthetic or naturally occurring in part. Barriers can include, for example, lipid bilayers having therein α-hemolysin, oligomeric protein channels such as porins, and synthetic peptides and the like. Barriers can also include inorganic plates having one or more holes of a suitable size.

It will be appreciated that in the embodiment where more than one nanopore is present then analysis of the target nucleic acid will occur by way of parallel analyses of the more than one nanpore.

In one embodiment the first target region is a gene or chromosome the relative copy number of which is to be studied, and the second target region is a different gene or chromosome present in normal copy number.

It will be appreciated that the term "quantitative difference" used herein refers to a difference in the number of copies of a gene, operon, chromosome, part of a chromosome, or a nucleic acid sequence, such as an amplification product. The term "quantitative detection" used herein refers to the detection of the difference in the number of copies of a gene, operon, chromosome, part of a chromosome, or a nucleic acid sequence, such as an amplification product.

In one embodiment, the detection provides prenatal diagnosis of a fetal chromosome imbalance or abnormality. The uses (and methods) of the invention have the advantage that a non-invasive procedure can be used for a diagnosis of a gene or chromosome abnormality in a fetus using, for example, a maternal blood sample. Thus, carrying out the method of the invention does not increase the risk of termination of a pregnancy.

In one embodiment, the detection step comprises a nanopore detection method which may be performed in accordance with the methodology described in Singer et al (2010) Nano Lett. 10, 738-742. In one embodiment, the detection step comprises the use of a detector which comprises a single nanopore of less than 10 nm in diameter, such as less than 5 nm in diameter (in particular approximately 4 to 5 nm in diameter). Such a diameter range is advantageous because it allows entry of unfolded DNA and slower transport than larger pores which provides more accurate detection of the passing of probes. The nanopore itself is fabricated in a free-standing silicon nitride (SiN) membrane using a focused electron beam. The detector chip may be constructed between two miniature fluid chambers (referred to as cis and trans) which are hydrated using a buffered 1M KCl solution. A positive bias is then applied across the SiN membrane using a pair of Ag/AgCl electrodes. Negatively charged DNA molecules are then captured and linearly threaded through the nanopore from the cis to trans chambers. During the threading process, the ion current is transiently reduced to a value that reflects to a first approximation the displacement of electrolytes from the nanopore by the DNA segment in the pore. The nanopore detection method, which finds excellent utility in the uses and methods of the invention, allows the high-throughput (>1 molecule/second at sub nM DNA concentrations) identification of specific nucleic acid sequences using hybridized probes.

In one embodiment, the detection step comprises the methodology described in WO 2010/004265 which is related to the nanopore detection method described herein with the additional covalent attachment of a transmembrane protein pore subunit to a nucleic acid handling enzyme, which results in a construct that is capable of both forming a pore and handling nucleic acids. The fixed nature and close proximity of the enzyme to the pore results in a proportion of the nucleotides in a target nucleic acid interacting with the pore and affect the current flowing through the pore in a distinctive manner to create the specific signature required by the invention to differentiate a first target region from a second target region.

In one embodiment, the detection step comprises the methodology described in WO 2007/041621 which uses a nanopore in a manner that allows the detection of the positions (relative and/or absolute) of nucleic acid probes that are hybridized onto a single-stranded nucleic acid molecule of interest. As the strand of interest and hybridized probes translocate through the nanopore, the fluctuations in current measured across the nanopore will vary as a function of time. These fluctuations in current are then used to determine the attachment positions of the probes along the strand of interest.

In one embodiment, the detection step comprises the methodology described in WO 2010/080617 which employs membranes having a nanopore with a focused and localized electric field through which a DNA molecule can translocate from a compartment adjacent to one membrane surface, through the membrane's pore, and out the opposing membrane surface adjacent a second compartment, to facilitate DNA analysis, sorting and detection.

In one embodiment, the detection step comprises the methodology described in WO 2009/155423 which comprises a long, thin analyte chamber having a nanopore or small number of nanopores formed therein within an interaction region. More specifically, the analyte chamber has a length approximately 100 times the width and height of the chamber, with the interaction region located at a specific distance from the second electrode. A sample including an analyte of interest is introduced into the analyte chamber through an access port. First and second electrodes are positioned on either side of the interaction region and provide an electric field along the length of the analyte chamber. This field causes an electrophoretic force that drives the target molecules or analytes of interest toward the nanopore in order to increase the number of target molecules in the vicinity of the nanopore (i.e., the interaction region) and, thereby, increase the number of molecules per unit time that interact with the nanopore. The analyte of interest may then be measured or analyzed utilizing pore blocking signals detected by a current sensor.

In one embodiment, the detection step comprises the methodology described in US 2009/298072 which specifically relates to the comparison of one given signature obtained by passing DNA through a pore of suitable diameter by applying an electric field to the DNA with another given signature to determine the identity of such a DNA molecule.

In one embodiment, the detection step comprises the methodology described in WO 2009/007743 which uses nanopore recordings to detect nucleic acid molecules. When added to a reservoir, nucleic acid strands are electrophoretically driven through the pore. The temporary blockade of the pore leads to a reversible reduction in the current flowing through the pore. Given that the current is flowing through a single channel, nanopore recordings are capable of detecting individual nucleic acid strands.

In one embodiment, the detection step comprises the methodology described in Clarke, J. et al (2009) Nature Nanotechnology 4, 265-270 which additionally comprises a covalently attached adapter molecule to continuously identify unlabeled nucleoside 5'-monophosphate molecules with accuracies averaging 99.8%.

Suitable examples of nucleic acid include may be DNA or RNA, particularly DNA. When the nucleic acid comprises DNA, the DNA may be single or double-stranded. The nucleic acid may be a mixture of nucleic acid from malignant and normal/non-malignant tissue. The nucleic acid may be a mixture of nucleic acid from malignant and normal/non-malignant tissue present in a biopsy or body fluid sample. The nucleic acid may be a mixture of maternal and fetal nucleic acid. The nucleic acid may be a mixture of maternal and fetal nucleic acid found in a maternal blood sample.

The sample may be a tissue sample, such as a biopsy or tissue explant. The sample may comprise blood. The sample may, in one embodiment, comprise maternal blood. The sample may comprise body fluid. The sample may comprise blood plasma or serum. The sample may be maternal blood plasma, which comprises fetal nucleic acid. The sample may comprise maternal and fetal derived nucleic acid. The sample may be collected by a non-invasive procedure with respect to the fetus and/or the amniotic sac. The sample may be collected intravenously from a pregnant woman.

The sample may be obtained from amniotic fluid surrounding a fetus or embryo *in utero,* or directly from the fetus or embryo *in utero.* The sample may be obtained from a mammalian subject, preferably the sample is from a human.

Although highly effective, it will be appreciated that the single molecule analysis of the known nanopore detection methods will generate a large amount of data which will require analysis and diagnosis by bioinformaticians. Therefore, there is a need to provide a reliable and effective method of detecting quantitative differences between nucleic acid samples which is simpler, cheaper and without the burden of analysing large amounts of data.

Thus, according to an aspect of the invention, there is provided a method for the detection of a quantitative difference between the amount of a first target region of nucleic acid and a second target region of nucleic acid in a sample, such that the first target region is a gene or chromosome the relative copy number of which is to be studied, and the second target region is a different gene or chromosome present in normal copy number, wherein said method comprises the steps of:
(a) providing the sample comprising the nucleic acid;
(b) hybridisation of one or more first probes to the first target region;
(c) hybridisation of one or more second probes to the second target region, such that the number and/or positioning of second probes on the second target region differs from the number and/or positioning of first probes on the first target region;
(d) detection of the number and/or position of the first probes identified on nucleic acid molecules within the sample as a specific signature assignable to the presence of the first target region;
(e) detection of the number and/or position of the second probes identified on nucleic acid molecules within the sample as a specific signature assignable to the presence of the second target region;

wherein a difference between the number of specific signatures obtained in step (d) and the number of specific signatures obtained in step (e) is indicative of a quantitative difference between the amount of the first and second target regions of nucleic acid in the sample; and
wherein the detection steps (d) and (e) comprise a nanopore based detection method.

According to a further aspect of the invention, there is provided a method for detection of an abnormality in a gene or chromosome copy number in a sample, comprising the steps of:
(a) providing the sample comprising the nucleic acid;
(b) hybridisation of one or more first probes to the first target region;
(c) hybridisation of one or more second probes to the second target region, such that the first target region is a gene or chromosome the relative copy number of which is to be studied, and the second target region is a different gene or chromosome present in normal copy number, and such that the number and/or positioning of second probes on the second target region differs from the number and/or positioning of first probes on the first target region;
(d) detection of the number and/or position of the first probes identified on nucleic acid molecules within the sample as a specific signature assignable to the presence of the first target region;
(e) detection of the number and/or position of the second probes identified on nucleic acid molecules within the sample as a specific signature assignable to the presence of the second target region;

wherein a difference between the number of specific signatures obtained in step (d) and the number of specific signatures obtained in step (e) is indicative of a quantitative difference between the amount of the first and second target regions of nucleic acid in the sample, and wherein the detection of a quantitative difference is indicative of an abnormality in a gene or chromosome copy number; and
wherein the detection steps (d) and (e) comprise a nanopore based detection method.

The invention of these aspects therefore provides a more targeted approach of directly comparing the overall signatures generated by nucleic acids as a whole within a sample rather than analysing the nucleic acids at the single nucleotide level. These aspects of the invention therefore provide methods which generate far less data and therefore significantly improve the efficiency of the diagnostic procedure.

In one embodiment, the one or more first probes and/or the one or more second probes comprise peptide nucleic acid (PNA), bis-PNA or DNA, such as single stranded DNA (ssDNA). In a further embodiment, the one or more first probes and/or the one or more second probes comprise PNA or bis-PNA. Peptide nucleic acids (PNAs) are a prominent class of artificial nucleic acid analogs which are netural oligomers with a peptide-like backbone onto which nucleobases are grafted in a designed sequence. Bis-PNA molecules consist of two PNA oligomers connected by a flexible linker (Singer et al (2010) Nano Lett. 10, 738-742). In a yet further embodiment, the one or more first probes and/or the one or more second probes comprise bis-PNA or a variant thereof (such as pseudocomplementary PNA or y-PNA). Without being bound by theory, it is believed that bis-PNA binds to one of the two strands of the target region of nucleic acid with high affinity and with sequence specificity by virtue of Watson-Crick and Hoogsteen base pairs. It will be appreciated that hybridization of the one or more first probes and/or the one or more second probes will be carried out in accordance with known methodology.

In one embodiment, the one or more first probes and/or the one or more second probes comprise one or more oligonucleotides, such as a 10 to 25 base pair oligonucleotide, in particular a 10 to 15 base pair oligonucleotide.

In one embodiment, the one or more first probes and/or the one or more second probes comprise a detectable tag or label. The presence of such a tag or label will greatly enhance the ability of the detection steps (d) and (e) to be able to detect the signature assigned to the first and second target regions. Such a tag or label may be incorporated within the probe during amplification. Examples of suitable tags or labels include fluorescence, magnetic or radioactive molecules and/or particles. Examples of suitable fluorophores include FAM - 6 carboxyfluorescein or TET - tetrachlorofluorescein.

Also disclosed is when the detection step of steps (d) and (e) comprises a flow cytometry based detection method, a piezoelectric based detection method or a digital PCR based detection method.

Digital PCR involves multiple PCR analyses on extremely dilute nucleic acids such that most positive amplifications reflect the signal from a single template molecule, permitting the counting of individual template molecules. The proportion of positive amplifications among the total number of PCRs analyzed allows an estimation of the template concentration in the original nondiluted sample. The process of digital PCR has been demonstrated to be of particular utility in the molecular detection of fetal chromosomal aneuploidy (Lo et al, (2007) PNAS 104(32), 13116-13121).

For flow cytometric analysis, cells or cell fragments are suspended in solution and are stained with fluorescent dyes. In the flow cytometer, cells are forced in a narrow stream through the path of an intense light source, such as a laser. The particles pass the laser beam in single file at a rate of several thousand per second. When the cells enter the light spot, they scatter light or emit fluorescence. As each particle passes through the light source, its optical properties are quantified and stored. Large numbers of cells can be measured individually, and the optical properties of a cell population can be determined in a short time. Many flow cytometers also have the ability to sort cells. For this purpose, the sample stream is broken into droplets after the point where the optical measurements are performed. Droplets containing desired cells are given an electric charge and are deflected into a collection tube by the influence of an electrostatic field.

Although the technique of flow cytometry is commonly used for the measurement and detection of cells, one of the key applications of flow cytometry is the detection and measurement of DNA and for the avoidance of doubt it will be appreciated that the methods of the invention requirement flow cytometry to analyse DNA rather than cells.

In one particular embodiment, the detection step of steps (d) and (e) comprises a nanopore based detection method as hereinbefore defined. Without being bound by theory, a nucleotide in a molecule of DNA passing from a nanopore in a membrane that has a low current applied to it will leave a specific signature creating a differential potential. These signals are recorded and a nucleotide sequence is generated. Commonly available nanopore detection methods are able to recognise double-stranded versus single-stranded DNA molecules or triple-stranded (if the hybridisation marker is a PNA molecule) versus double-stranded DNA molecules. The advantage of such a technique resides in the speed and the low quantity of material analysed.

Also disclosed is when the detection step of steps (d) and (e) comprise the use of a piezoelectric plate detection method, such as a bead-based hybridization extraction method, using a piezoelectric plate detection method for quantification of analyte. Without being bound by theory, oligonucleotide binders may be used as capture agents to bind the target oligonucleotide in the sample, and extract it for analysis. The oligonucleotide binders may themselves be bound to beads or to biotin or other molecules (including antibodies) to assist in their role as capture agents.

If using magnetic beads, the number of magnetic beads bound, and hence concentration of the analyte (target oligonucleotide) in the sample, can be determined through their adherence to a piezoelectric plate, with consequent change in the frequency of its vibration which can be electronically detected with great accuracy. Examples of suitable piezoelectric detection methodology may be found within US 2009/293590, US 2009/168068, WO 2009/035732, US 2009/282902, WO 2008/019694, US 2006/105471, US 2006/014270, US 2005/186684, WO 2004/001392, WO 2004/001417, WO 2004/001416, WO 01/26138, US 6,161,426, US 5,447,845, WO 2006/119308, WO 2007/030155 and UK Patent Application No. 1002627.6.

In one embodiment, the number of second probes differs from the number of first probes. Such a difference in number will enable the first target region to be differentiated from the second target region by virtue of a specific signature of probe measurements associated with the first target region which will differ in number from the specific signature of probe measurements associated with the second target region. For example, using the nanopore based detection method described herein before, the second probe will create a given number of signal peaks characteristic of the presence of the given number of second probes hybridized to the second target region when passing the sample through the nanopore. In addition, the first probe will create a differing number of signal peaks characteristic of the presence of a different number of first probes hybridized to the first target region when passing the sample through the nanopore. The number of probes on each target region therefore comprises a specific signature very much akin to a "barcode" which will immediately allow the differentiation between the presence of a first target region from a second target region.

In one embodiment, the position of second probes differs from the position of first probes. Such a difference in distance between each of the probes will enable the first target region to be differentiated from the second target region by virtue of a specific profile of probe measurements associated with the first target region which will differ in profile from the specific signature of probe measurements associated with the second target region. For example, using the nanopore based detection method described hereinbefore, the second probe will create a given number of signal peaks characteristic of the distance between the given number of second probes hybridized to the second target region when passing the sample through the nanopore. In addition, the first probe will create a differing number of signal peaks characteristic of the presence of a different distance between the first probes hybridized to the first target region when passing the sample through the nanopore. As discussed hereinbefore, the distance of probes on each target region therefore comprises a barcode which will immediately allow the differentiation between the presence of a first target region from a second target region.

In one embodiment, the number and position of second probes differs from the number and position of first probes. Such a difference in number and distance between each of the probes will enable the first target region to be differentiated from the second target region by virtue of a specific profile of probe measurements associated with the first target region which will differ in profile from the specific signature of probe measurements associated with the second target region. For example, using the nanopore based detection method described hereinbefore, the second probe will create a given number of signal peaks characteristic of the number and distance between the given number of second probes hybridized to the second target region when passing the sample through the nanopore. In addition, the first probe will create a differing number of signal peaks characteristic of the presence of a different number and distance between the first probes hybridized to the first target region when passing the sample through the nanopore. As discussed hereinbefore, the number and distance of probes on each target region therefore comprises a barcode which will immediately allow the differentiation between the presence of a first target region from a second target region.

References herein to the term "signature" or "barcode", when applied to the nanopore detection method, refer to the profile obtained from a nucleotide passing through a pore via application of an electronic field. Such a profile may include, for example, the duration of the nucleotide's passage through the pore together with the observed amplitude of current during that passage. Electronic signatures can be visualized, for example, by a plot of current (e.g. pA) versus time. Electronic signature for a DNA is also envisioned and can be, for example, a plot of current (e.g. pA) versus time for the DNA to pass through the pore via application of an electric field.

The invention has an advantage in that a very small difference in gene or chromosome copy number can be detected. The invention may increase the sensitivity of quantitative detection of nucleic acid relative to existing PCR amplification methods by detecting each and every probe containing molecule and being able to differentiate first probe containing molecules from second probe containing molecules to easily and accurately provide a sensitive measure of an excess copy number.

The method advantageously may be applied to detecting quantitative differences in DNA sequence present in different groups of somatic cells within the same organism or in prenatal diagnosis of hereditary conditions.

The invention has an advantage in that the method is not dependent on SNPs or epigenetic modifications, and is hence not limited to a select percentage of the population.

An abnormality in a chromosome number may be an additional copy of a whole or part of a chromosome, or a missing copy of a whole or part of a chromosome. An additional copy number of a chromosome may be two or more copies, or three or more copies, of the chromosome or part of the chromosome, i.e. where the chromosome or part of the chromosome is in triplicate (also known in the art as a "trisomy"). For example, there are three copies of chromosome 21 in a subject with Down's Syndrome compared with two copies in a subject without Down's Syndrome. A missing copy of a chromosome may result in a single copy or no copy, of a chromosome instead of the normal two copies in a healthy/un-afflicted subject (or one copy in the case of sex chromosomes in males).

An abnormality in a gene copy number in a subject may be one or more additional copies of a gene relative to the average copy number of the gene in a sample of subjects of a general population. An abnormality in a gene copy number in a subject may be one or more reductions in copy number of a gene relative to the average copy number of the gene in a sample of subjects of a general population. The sample of subjects of a general population may comprise one or more individuals who do not have symptoms of a disease associated with the gene.

In one embodiment, the first target region of the nucleic acid is associated with an abnormality, such as a disease, and the second target region of the nucleic acid is used as a control/standard.

The first target region may be a part of a gene, operon, or chromosome which is associated with a disease, disability or other clinical syndrome and the second target region may be part of a gene, operon, or chromosome which is used as a control/standard, which is known to be present in normal copy number, or vice versa.

The first target region of nucleic acid may comprise at least part of a region of a human chromosome selected from any of the group comprising chromosome number 1, 2, 3, 4, 5, 6, 7, 8, 9, 10, 11, 12, 13, 14, 15, 16, 17, 18, 19, 20, 21, 22, 23, the X chromosome, and the Y chromosome. In one embodiment, the first target region of nucleic acid comprises a region of human chromosome selected from the group comprising human chromosome number 8, 9, 13, 16, 18, 21, and 22.

The first target region of nucleic acid may comprise a nucleic acid sequence of at least part of the HER2/neu (c-erbB-2) gene or at least part of the p53 gene. The first target region of nucleic acid may comprise a nucleic acid sequence of at least part of the *c-myc* gene, IL-6 gene, EGRF gene (Epidermal Growth Factor Receptor gene), BMI gene, or cadherin 7 gene. The first target region of nucleic acid may comprise a nucleic acid sequence of at least part of any of the group of genes comprising c-MYC, VEGFA, MMP9, PTEN, int-2/FGF3, KRAS, EBF1, IKZF1, GATA6, AKT2,MYB, SMAD4, CDKN2A, TOP2A, receptors for estrogen, progesterone, HER1, uPAR, uPA, MET, RET, GLI, AKT2, CCND1(cyclin D1), EGFR, ERBB2, MYCN, and MYCL1.

An advantage of the first target region of nucleic acid comprising a nucleic acid sequence of at least part of a specific gene, such as at least part of the HER2/neu (c-erbB-2) gene, is that mutations or changes in copy number of this gene may be detected. The HER2/neu (c-erbB-2) gene is implicated in some breast cancers. Meanwhile, the Myc family gene may be implicated in leukaemias, lung cancers, and breast cancers; the IL-6 gene and EGRF in neurological tumours; the BMI gene in lymphomas; and the cadherin 7 gene in prostate and testicular tumours.

The second target region of nucleic acid may comprise at least part of a region of a human chromosome of any of the group comprising chromosome number 1, 2, 3, 4, 5, 6, 7, 8, 9, 10, 11, 12, 13, 14, 15, 16, 17, 18, 19, 20, 21, 22, 23, the Q chromosome, and the Y chromosome. The second target region of nucleic acid may comprise a region of human chromosome selected from the group comprising chromosome 1, chromosome 2, chromosome 7, chromosome 9, chromosome 10, chromosome 11 and chromosome 14.

The second target region of nucleic acid may comprise a nucleic acid sequence of at least part of the housekeeping gene encoding glyceraldehyde-3-phosphate dehydrogenase (GAPDH) or Beta-actin.

The second target region of nucleic acid may comprise a nucleic acid sequence of at least part of a sequence that is normally in the same copy number as the first target region of nucleic acid. The second target region may be a highly conserved (for example, highly conserved in humans) sequence of nucleic acid. The second target region of nucleic acid may comprise a nucleic acid sequence of any single copy sequence or gene that is not present on the same chromosome as the first target region, for example, SLIT1 or PI3KADP1.

The first and/or second target regions of nucleic acid may be between about 10 and about 8000 base pairs long; or between about 20 and about 2000 base pairs long; or between about 50 and about 1000 base pairs long. The first and/or second target regions of nucleic acid may be less than 500 base pairs long, alternatively less than 300 base pairs long, and alternatively less than 200 base pairs long. The first and/or second target regions of nucleic acid may be between about 100 and about 200 base pairs long. In one embodiment, the first and second target regions of nucleic acid are similar in length, such as within 10%, alternatively within 20%, of each other.

In one embodiment, the first and second target sequences are different lengths.

The first and second target sequences may include different markers which allow them to be distinguished. For example, the first sequence may carry a first fluorophore and the second sequence may carry a second fluorophore, wherein the first and second fluorophore may be different.

In some cases only part of a chromosome is in excess copy number (e.g. partial trisomy) or in reduced copy number (e.g. deletion or monosomy), thus, it is advantageous to select a particular region which is known to be in excess copy number or reduced copy number and causes symptoms of a chromosomal abnormality or related disease.

The detection of an abnormality in a chromosome copy number may comprise the detection of and/or diagnosis of a condition that is a hereditary numerical chromosome abnormality.

The detection of an abnormality in a chromosome copy number may comprise the detection of and/or diagnosis of a condition selected from the group comprising Down's Syndrome (Trisomy 21), Edward's Syndrome (Trisomy 18), Patau syndrome (Trisomy 13), Trisomy 9, Warkany syndrome (Trisomy 8), Cat Eye Syndrome (4 copies of chromosome 22), Trisomy 22, and Trisomy 16. Additionally, or alternatively, the detection of an abnormality in a gene, chromosome, or part of a chromosome, copy number may comprise the detection of and/or diagnosis of a condition selected from the group comprising Wolf-Hirschhorn syndrome (4p-), Cri du chat syndrome (5p-), Williams-Beuren syndrome (7-), Jacobsen Syndrome (11-), Miller-Dieker syndrome (17-), Smith-Magenis Syndrome (17-), 22q11.2 deletion syndrome (also known as Velocardiofacial Syndrome, DiGeorge Syndrome, conotruncal anomaly face syndrome, Congenital Thymic Aplasia, and Strong Syndrome), Angelman syndrome (15-), and Prader-Willi syndrome (15-).

Additionally, or alternatively, the detection of an abnormality in the chromosome copy number may comprise the detection of and/or diagnosis of a condition selected from the group comprising Turner syndrome (Ullrich-Turner syndrome or monosomy X), Klinefelter's syndrome, 47,XXY or XXY syndrome, 48,XXYY syndrome, 49 XXXXY Syndrome, Triple 25 X syndrome, XXXX syndrome (also called tetrasomy X, quadruple X, or 48, XXXX), XXXXX syndrome (also called pentasomy X or 49, XXXXX), and XYY syndrome.

Additionally, or alternatively, the detection of an abnormality in the gene or chromosome copy number may comprise the detection of and/or diagnosis of a condition selected from any of the group listed in Table 1:

**Table 1 Chromosome Abnormalities and Disease**

| **Chromosome Abnormality** | | **Disease Association** |
|---|---|---|
| X,Y | XO | Turner's syndrome |
| | XXY | Klinefelter syndrome |
| | XYY | Double Y syndrome |
| | XXX | Trisomy X syndrome |
| | XXXX | Four X syndrome |
| | Xp21 deletion | Duchenne's/Becker syndrome, congenital adrenal hypoplasia, chronic granulomatus disease |
| | Xp22 deletion | steroid sulfatase deficiency |
| | Xq26 deletion | X-linked lymphproliferative disease |
| 1 | 1p- (somatic) | neuroblastoma |
| | monosomy | |
| | trisomy | |
| 2 | monosomy | |
| | trisomy 2q | growth retardation, developmental and mental delay, and minor physical abnormalities |
| 3 | monosomy | |
| | trisomy (somatic) | non-Hodgkin's lymphoma |
| 4 | monosomy | |
| | trisomy (somatic) | Acute non lymphocytic leukaemia (AN LL) |
| 5 | 5p- | Cri du chat; Lejeune syndrome |
| | 5q- (somatic) | Myelodysplastic syndrome |
| | monosomy | |
| | trisomy | |
| 6 | monosomy | |
| | trisomy (somatic) | clear-cell sarcoma |
| 7 | 7q11.23 deletion | William's syndrome |
| | monosomy | monosomy 7 syndrome of childhood; somatic: renal cortical adenomas; myelodysplastic syndrome |
| | trisomy | |
| 8 | 8q24.1 deletion | Langer-Giedon syndrome |
| | monosomy | |
| | trisomy | myelodysplastic syndrome; Warkany syndrome; somatic: chronic myelogenous leukaemia |
| 9 | monsomy 9p | Alfi's syndrome |
| | monosomy | |
| | 9p partial trisomy | Rethore syndrome |
| | trisomy | complete trisomy 9 syndrome; mosaic trisomy 9 syndrome |
| 10 | manosomy | |
| | trisomy (somatic) | ALL or ANLL |
| 11 | 11p- | Aniridia; Wilms tumor |
| | 11q- | Jacobson Syndrome, |
| | monosomy (somatic) | myeloid lineages effected (ANLL, MDS) |
| | trisomy | |
| 12 | monosomy | |
| | Trisomy (somatic) | CLL, Juvenile granulosa cell tumor (JGCT) |
| 13 | 13q- | 13q- syndrome; Orbeli syndrome |
| | 13q14 deletion | retinoblastoma |
| | monosomy | |
| | trisomy | Patau's syndrome |
| 14 | monsomy | |
| | trisomy (somatic) | myeloid disorders (MDS, ANLL, atypical CML) |
| 15 | 15q11-q13 deletion | Prader-Willi, Angelman's syndrome |
| | monosomy | |
| | trisomy (somatic) | myeloid and lymphoid lineages affected, e.g., MDS, ANLL, ALL, CLL) |
| 16 | 16q13.3 deletion | Rubenstein-Taybi |
| | monosomy | |
| | trisomy (somatic) | papillary renal call carcinomas (malignant) |
| 17 | 17p- (somatic) | 17p syndrome in myeloid malignancies |
| | 17q11.2 deletion | Smith-Magenis |
| | 17q13.3 | Miller-Dieker |
| | monosomy | |
| | trisomy (somatic) | renal cortical adenomas |
| | 17p11.2-12 trisomy | Chareot-Marie Tooth Syndrome type 1;HNPP |
| 18 | 18p- | 18p partial monosomy Syndrome or Grouchy Lamy Thieffry syndrome |
| | 18q- | Grouchy Lamy Salmon Laundry Syndrome |
| | monosomy | |
| | Trisomy | Edwards Syndrome |
| 19 | monosomy | |
| | trisomy | |
| 20 | 20p- | trisomy 20p syndrome |
| | 20p11.2-12 deletion | Alagille |
| | 21q- | somatic: MDS, ANLL, polycythemia vera, chronic neutrophilic leukemia |
| | monosomy | |
| | trisomy (somatic) | papillary renal cell carcinomas (malignant) |
| 21 | monosomy | |
| | trisomy | Down's syndrome |
| 22 | 22q11.2 deletion | DiGeorge's syndrome, velocardiofacial syndrome, conotruacal anomaly face syndrome, autosomal dominant Opitz G/BBB syndrome, Caylor cardiofacial syndrome |
| | monosomy | |
| | trisomy | complete trisomy 22 syndrome |

The detection of an abnormality in a gene copy number may comprise the detection of and/or diagnosis of a cancer related condition.

The detection of an abnormality in a gene copy number may comprise the detection of and/or diagnosis of a condition selected from the group comprising breast cancer, leukaemia, lung cancer, neurological tumours, lymphomas, prostate cancer and testicular cancer.

Additionally, or alternatively, the detection of an abnormality in a gene copy number may comprise the detection of and/or diagnosis of a condition caused or associated with an additional copy number or reduced copy number of a gene.

The invention provides the use of the method of the invention, as described above, to determine if an individual has an increase or decrease in gene or chromosome copy number.

Also disclosed is a method of diagnosis to determine if an individual has an increase or decrease in gene or chromosome copy number by carrying out any method of the invention herein.

According to another aspect of the present invention, there is provided the use of any method of the invention to detect an abnormality in a gene or chromosome copy number in a sample for at least two different genes or chromosomes.

The method may be used to detect an abnormality in a gene or chromosome copy number for at least three, four, five, six or more different genes or chromosomes. An array, for example a multiwall array may be used.

According to another aspect of the invention, there is provided a use of any method according to the method herein, to determine if an individual has an increase or decrease in gene or chromosome copy number.

The term "diagnoses" used herein refers to the ability to demonstrate an increased likelihood that a subject has, or does not have, a specific condition or conditions or that an existing condition or conditions have certain specific characteristics such as therapy sensitivity/resistance.

Also disclosed is the use of the method of any other aspect of the invention to determine the choice of treatment for a condition.

The condition may be cancer. The choice of treatment may be a choice of chemotherapy regime and/or agent.

Also disclosed is a kit comprising one or more first probes and one or more second probes as herein defined suitable for carrying out the method according to the invention herein and instructions.

The invention will now be described with reference to the accompanying Figures:
The method of the invention may be performed by initially extracting cell-free DNA from a plasma sample obtained from a pregnant female human subject.

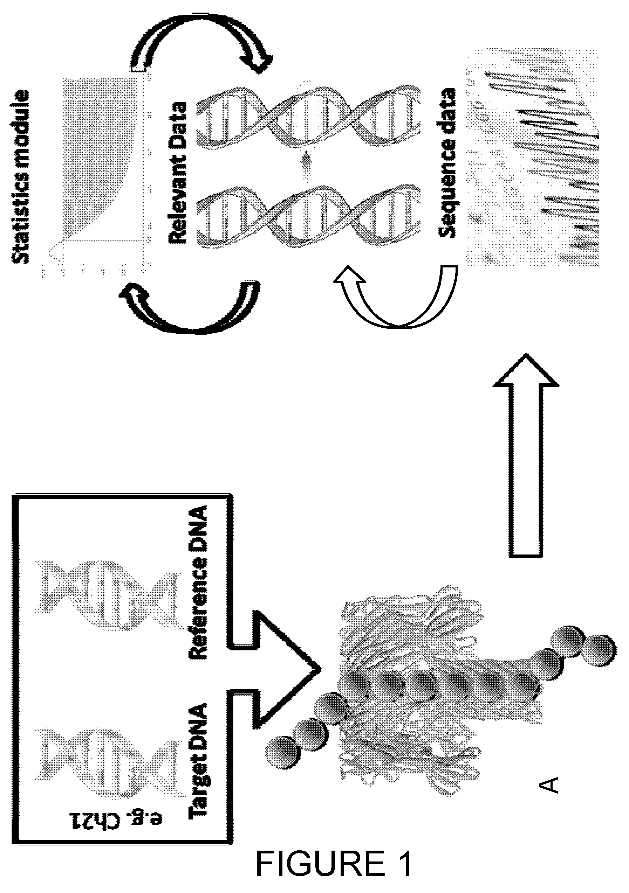

In accordance with the invention, the plasma DNA may then be suitably hybridised with DNA or PNA specific oligonucleotide probes shown as circles in Figure 1 which will be designed to leave a specific signature to the hybridised sample DNA (shown as "Target DNA, e.g. chromosome 21" and "Reference DNA" in Figure 1) that can be recognisable and quantifiable. The molecules of DNA may then be detected and counted by a mechanism (shown as "A" in Figure 1) which may typically comprise any of the methodology mentioned hereinbefore (i.e. nanopore technology) such that the target and reference DNA molecules will be counted and compared with each other in order to result in sequence data. The sequence data may then be used to generate relevant data such as relative DNA levels which have been found and the relevant data may be input into a statistics module which has the capacity to compare and set diagnostic thresholds on an iterative basis in order to arrive at a sensitive and effective diagnosis.

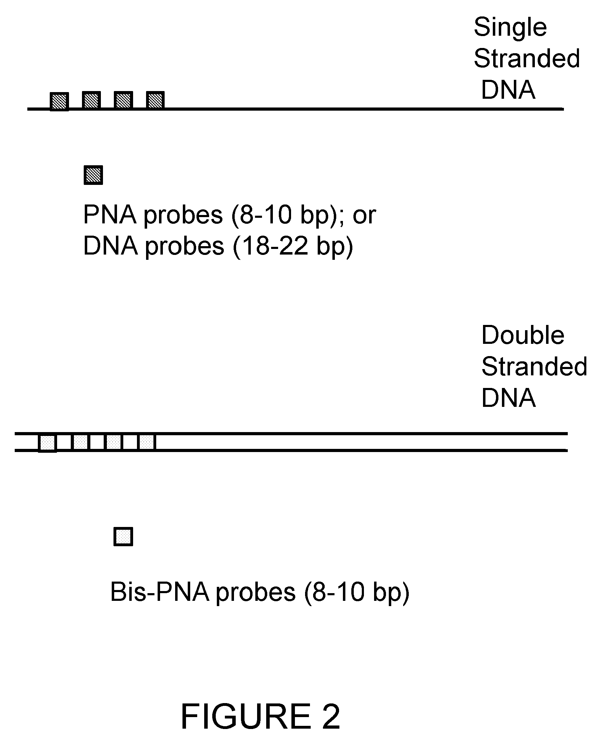

As shown in Figure 2, in accordance with the second aspect of the invention, the plasma DNA may be suitably hybridised with probes which may be bis-PNA (white squares in Figure 2) or PNA or DNA oligonucleotide probes (grey squares in Figure 2), which may additionally (in certain embodiments of the invention) carry covalent tags. These may be designed to hybridise with plasma DNA in native (double-stranded) form (forming triplex structures), or alternatively after denaturation to single-stranded form.

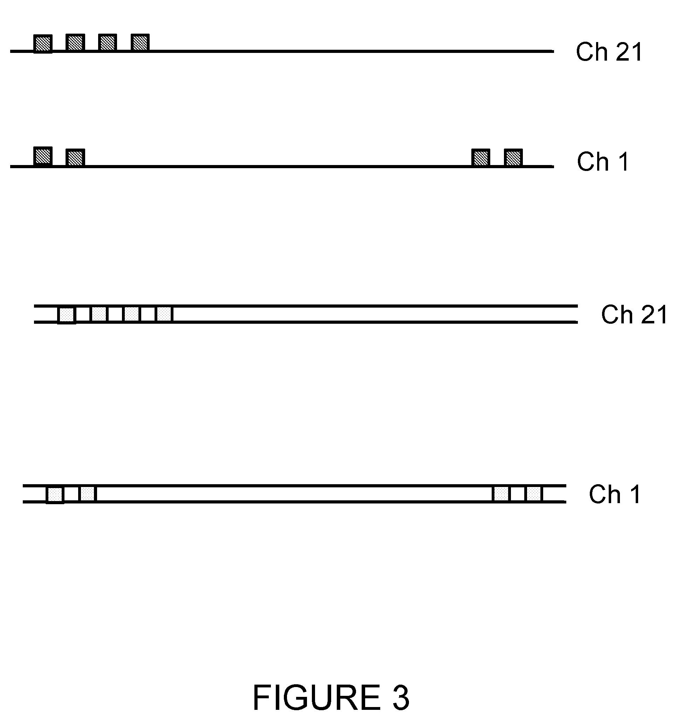

Figure 3 demonstrates one suitable application of the present invention for detecting trisomy 21 (i.e an excess of chromosome 21 (Ch 21) when compared with other chromosomes i.e. chromosome 1 (Ch 1)). For example, the upper panel in Figure 3 demonstrates 4 first DNA or PNA probes hybridised to a first target region (i.e. chromosome 21) which have specific and uniform spacing between the 4 first probes. The next panel in Figure 3 demonstrates 4 second DNA or PNA probes hybridised to a second target region (i.e. chromosome 1). It may be clearly seen from this panel in Figure 3 that the spacing of the probes hybridised to chromosome 1 differs from the probes hybridised to chromosome 21. The lower two panels of Figure 3 show the same arrangement of probes based on hybridisation of bis-PNA to double stranded DNA (dsDNA).

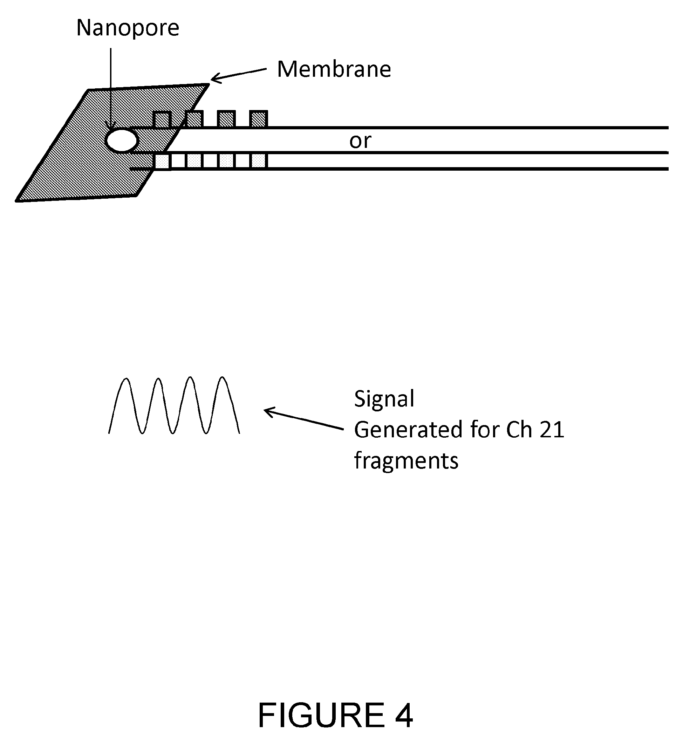

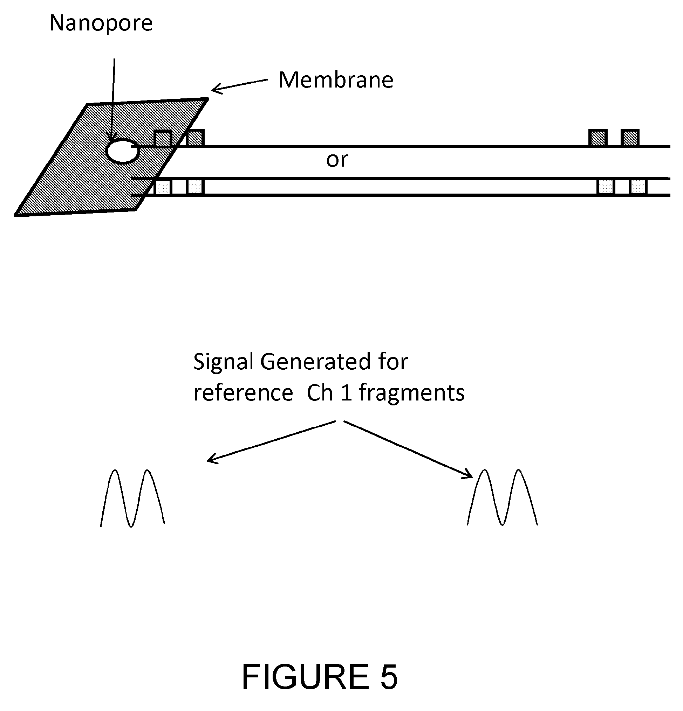

Figures 4 and 5 pictorially demonstrate how the counting of single molecules (DNA fragments) may be conducted based on the characteristic 'signature' generated by the spatial placement of attached probes along the DNA fragments which in turn generates a temporal code as the molecule passes through the nanopore device. For example, the signal obtained in Figure 4 is four uniformly spaced signal peaks corresponding to the specific signature or barcode representative of chromosome 21 (regardless of whether DNA, PNA or bis-PNA probes are used). By contrast, the signal obtained in Figure 5 is characteristic of two groups of two uniformly spaced signal peaks corresponding to the specific signature or barcode representative of chromosome 1 (once again, regardless of whether DNA, PNA or bis-PNA probes are used).

The final step of the procedure may typically comprise analysis of the resulting data obtained by molecule counting to quantify the molecules (DNA fragments) in excess. For example, a trisomy 21 pregnancy will be characterised by the presence of chromosome 21 in excess by comparison with one or more reference chromosomes such as chromosome 1.

## Claims

1. A method for the detection of a quantitative difference between the amount of a first target region of nucleic acid and a second target region of nucleic acid in a sample, such that the first target region is a gene or chromosome the relative copy number of which is to be studied, and the second target region is a different gene or chromosome present in normal copy number, wherein said method comprises the steps of:
(a) providing the sample comprising the nucleic acid;
(b) hybridisation of one or more first probes to the first target region;
(c) hybridisation of one or more second probes to the second target region, such that the number and/or positioning of second probes on the second target region differs from the number and/or positioning of first probes on the first target region;
(d) detection of the number and/or position of the first probes identified on nucleic acid molecules within the sample as a specific signature assignable to the presence of the first target region;
(e) detection of the number and/or position of the second probes identified on nucleic acid molecules within the sample as a specific signature assignable to the presence of the second target region;
wherein a difference between the number of specific signatures obtained in step (d) and the number of specific signatures obtained in step (e) is indicative of a quantitative difference between the amount of the first and second target regions of nucleic acid in the sample; and
wherein the detection steps (d) and (e) comprise a nanopore based detection method.

2. A method for detection of an abnormality in a gene or chromosome copy number in a sample, comprising the steps of:
(a) providing the sample comprising the nucleic acid;
(b) hybridisation of one or more first probes to the first target region;
(c) hybridisation of one or more second probes to the second target region, such that the first target region is a gene or chromosome the relative copy number of which is to be studied, and the second target region is a different gene or chromosome present in normal copy number, and such that the number and/or positioning of second probes on the second target region differs from the number and/or positioning of first probes on the first target region;
(d) detection of the number and/or position of the first probes identified on nucleic acid molecules within the sample as a specific signature assignable to the presence of the first target region;
(e) detection of the number and/or position of the second probes identified on nucleic acid molecules within the sample as a specific signature assignable to the presence of the second target region;
wherein a difference between the number of specific signatures obtained in step (d) and the number of specific signatures obtained in step (e) is indicative of a quantitative difference between the amount of the first and second target regions of nucleic acid in the sample, and wherein the detection of a quantitative difference is indicative of an abnormality in a gene or chromosome copy number; and
wherein the detection steps (d) and (e) comprise a nanopore based detection method.

3. A method as defined in claim 1 or claim 2, wherein the one or more first probes and/or the one or more second probes comprise peptide nucleic acid (PNA), bis-PNA or DNA, such as single stranded DNA (ssDNA).

4. A method as defined in any one of claims 1 to 3, wherein the one or more first probes and/or the one or more second probes comprise one or more oligonucleotides, such as a 10 to 25 base pair oligonucleotide, in particular a 10 to 15 base pair oligonucleotide.

5. A method as defined in any one of claims 1 to 4, wherein the one or more first probes and/or the one or more second probes comprise a detectable tag or label.

6. A method as defined in any one of claims 1 to 5, which comprises a single nanopore of less than 10 nm in diameter, such as less than 5 nm in diameter, in particular 4 to 5 nm in diameter.

7. A method as defined in any one of claims 1 to 6, wherein the nanopore comprises a silicon nitride (SiN) membrane.

8. A method as defined in any one of claims 1 to 7, wherein the sample is maternal blood plasma, which comprises fetal nucleic acid.

9. A method as defined in any one of claims 2 to 8, wherein the abnormality comprises three copies of chromosome 21 in a subject with Down's Syndrome compared with two copies in a subject without Down's Syndrome.

10. Use of a method as defined in any one of claims 1 to 9, to determine if an individual has an increase or decrease in gene or chromosome copy number.

## Patentansprüche

1. Verfahren zum Detektieren einer quantitativen Differenz zwischen der Menge einer ersten Targetregion von Nucleinsäure und einer zweiten Targetregion von Nucleinsäure in einer Probe, so dass die erste Targetregion ein Gen oder Chromosom ist, dessen relative Kopiezahl zu untersuchen ist, und die zweite Targetregion ein anderes Gen oder Chromosom ist, das mit normaler Kopiezahl vorliegt, wobei das genannte Verfahren die folgenden Schritte beinhaltet:
(a) Bereitstellen der Probe, die die Nucleinsäure beinhaltet;
(b) Hybridisieren von einer oder mehreren ersten Sonden mit der ersten Targetregion;
(c) Hybridisieren von einer oder mehreren zweiten Sonden mit der zweiten Targetregion, so dass sich Anzahl und/oder Positionierung von zweiten Sonden auf der zweiten Targetregion von Anzahl und/oder Positionierung von ersten Sonden auf der ersten Targetregion unterscheidet;
(d) Detektieren von Anzahl und/oder Position der auf Nucleinsäuremolekülen in der Probe identifizierten ersten Sonden als eine spezifische Signatur, die der Anwesenheit der ersten Targetregion zuschreibbar ist;
(e) Detektieren von Anzahl und/oder Position der auf Nucleinsäuremolekülen in der Probe identifizierten zweiten Sonden als eine spezifische Signatur, die der Anwesenheit der zweiten Targetregion zuschreibbar ist;
wobei eine Differenz zwischen der Anzahl der in Schritt (d) erhaltenen spezifischen Signaturen und der Anzahl der in Schritt (e) erhaltenen spezifischen Signaturen auf eine quantitative Differenz zwischen der Menge der ersten und zweiten Targetregionen von Nucleinsäure in der Probe hindeutet; und
wobei die Detektionsschritte (d) und (e) ein Detektionsverfahren auf der Basis von Nanoporen beinhalten.

2. Verfahren zum Detektieren einer Anomalie bei einer Gen- oder Chromosomen-Kopiezahl in einer Probe, das die folgenden Schritte beinhaltet:
(a) Bereitstellen der Probe, die die Nucleinsäure beinhaltet;
(b) Hybridisieren von einer oder mehreren ersten Sonden mit der ersten Targetregion;
(c) Hybridisieren von einer oder mehreren zweiten Sonden mit der zweiten Targetregion, so dass die erste Targetregion ein Gen oder Chromosom ist, dessen relative Kopiezahl zu untersuchen ist, und die zweite Targetregion ein anderes Gen oder Chromosom ist, das mit normaler Kopiezahl vorliegt, so dass sich Anzahl und/oder Positionierung von zweiten Sonden auf der zweiten Targetregion von Anzahl und/oder Positionierung von ersten Sonden auf der ersten Targetregion unterscheidet;
(d) Detektieren von Anzahl und/oder Position der auf Nucleinsäuremolekülen in der Probe identifizierten ersten Sonden als eine spezifische Signatur, die der Anwesenheit der ersten Targetregion zuschreibbar ist;
(e) Detektieren von Anzahl und/oder Position der auf Nucleinsäuremolekülen in der Probe identifizierten zweiten Sonden als eine spezifische Signatur, die der Anwesenheit der zweiten Targetregion zuschreibbar ist;
wobei eine Differenz zwischen der Anzahl der in Schritt (d) erhaltenen spezifischen Signaturen und der Anzahl der in Schritt (e) erhaltenen spezifischen Signaturen auf eine quantitative Differenz zwischen der Menge der ersten und zweiten Targetregionen von Nucleinsäure in der Probe hindeutet, und wobei die Detektion einer quantitativen Differenz auf eine Anomalie bei einer Gen- oder Chromosomen-Kopiezahl hindeutet; und
wobei die Detektionsschritte (d) und (e) ein Detektionsverfahren auf der Basis von Nanoporen beinhalten.

3. Verfahren nach Anspruch 1 oder Anspruch 2, wobei die eine oder mehreren ersten Sonden und/oder die eine oder mehreren zweiten Sonden Peptidnucleinsäure (PNA), bis-PNA oder DNA, wie eine einzelsträngige DNA (ssDNA), beinhalten.

4. Verfahren nach einem der Ansprüche 1 bis 3, wobei die eine oder mehreren ersten Sonden und/oder die eine oder mehreren zweiten Sonden ein oder mehrere Oligonucleotide wie ein 10 bis 25 Basenpaar-Oligonucleotid, insbesondere ein 10 bis 15 Basenpaar-Oligonucleotid, beinhalten.

5. Verfahren nach einem der Ansprüche 1 bis 4, wobei die eine oder mehreren ersten Sonden und/oder die eine oder mehreren zweiten Sonden ein(e) detektierbare(s) Tag oder Markierung beinhalten.

6. Verfahren nach einem der Ansprüche 1 bis 5, das eine einzelne Nanopore mit einem Durchmesser von weniger als 10 nm, z.B. mit einem Durchmesser von weniger als 5 nm, insbesondere mit einem Durchmesser von 4 bis 5 nm, beinhaltet.

7. Verfahren nach einem der Ansprüche 1 bis 6, wobei die Nanopore eine Siliziumnitrid-(SiN)-Membran beinhaltet.

8. Verfahren nach einem der Ansprüche 1 bis 7, wobei die Probe mütterliches Blutplasma ist, das fötale Nucleinsäure beinhaltet.

9. Verfahren nach einem der Ansprüche 2 bis 8, wobei die Anomalie drei Kopien von Chromosom 21 in einem Subjekt mit Down-Syndrom im Vergleich zu zwei Kopien in einem Subjekt ohne Down-Syndrom beinhaltet.

10. Verwendung eines Verfahrens nach einem der Ansprüche 1 bis 9 zum Bestimmen, ob ein Individuum eine Erhöhung oder Verringerung der Gen- oder Chromosomen-Kopiezahl aufweist.

## Revendications

1. Procédé de détection d'une différence quantitative entre les quantités d'une première région cible d'un acide nucléique et d'une deuxième région cible d'un acide nucléique dans un échantillon, tel que la première région cible est un gène ou un chromosome dont le nombre relatif de copies est à étudier et la deuxième région cible est un gène ou un chromosome différent présent à un nombre de copies normal, ledit procédé comprenant les étapes qui consistent à:
(a) se munir de l'échantillon comprenant l'acide nucléique;
(b) hybrider une ou plusieurs premières sondes à la première région cible ;
(c) hybrider une ou plusieurs deuxièmes sondes à la deuxième région cible de sorte que le nombre et/ou la position des deuxièmes sondes sur la deuxième région cible diffère(nt) du nombre et/ou de la position des premières sondes sur la première région cible ;
(d) détecter le nombre et/ou la position des premières sondes identifiées sur des molécules d'acide nucléique dans l'échantillon en tant que signature spécifique assignable à la présence de la première région cible ;
(e) détecter le nombre et/ou la position des deuxièmes sondes identifiées sur des molécules d'acide nucléique dans l'échantillon en tant que signature spécifique assignable à la présence de la deuxième région cible ;
dans lequel une différence entre le nombre de signatures spécifiques obtenues à l'étape (d) et le nombre de signatures spécifiques obtenues à l'étape (e) est indicatrice d'une différence quantitative entre les quantités des première et deuxième régions cibles d'un acide nucléique dans l'échantillon ; et
dans lequel les étapes de détection (d) et (e) comprennent une méthode de détection reposant sur un nanopore.

2. Procédé de détection d'une anomalie dans le nombre de copies d'un gène ou d'un chromosome dans un échantillon, comprenant les étapes qui consistent à:
(a) se munir de l'échantillon comprenant l'acide nucléique;
(b) hybrider une ou plusieurs premières sondes à la première région cible ;
(c) hybrider une ou plusieurs deuxièmes sondes à la deuxième région cible, le procédé étant tel que la première région cible est un gène ou un chromosome dont le nombre relatif de copies est à étudier et la deuxième région cible est un gène ou un chromosome différent présent à un nombre de copies normal et tel que le nombre et/ou la position des deuxièmes sondes sur la deuxième région cible diffère(nt) du nombre et/ou de la position des premières sondes sur la première région cible ;
(d) détecter le nombre et/ou la position des premières sondes identifiées sur des molécules d'acide nucléique dans l'échantillon en tant que signature spécifique assignable à la présence de la première région cible ;
(e) détecter le nombre et/ou la position des deuxièmes sondes identifiées sur des molécules d'acide nucléique dans l'échantillon en tant que signature spécifique assignable à la présence de la deuxième région cible ;
dans lequel une différence entre le nombre de signatures spécifiques obtenues à l'étape (d) et le nombre de signatures spécifiques obtenues à l'étape (e) est indicatrice d'une différence quantitative entre les quantités des première et deuxième régions cibles d'un acide nucléique dans l'échantillon ; et dans lequel la détection d'une différence quantitative est indicatrice d'une anomalie dans le nombre de copies du gène ou chromosome ; et
dans lequel les étapes de détection (d) et (e) comprennent une méthode de détection reposant sur un nanopore.

3. Procédé selon la revendication 1 ou la revendication 2, dans lequel la ou les plusieurs premières sondes et/ou la ou les plusieurs deuxièmes sondes comprennent un acide nucléique peptidique (PNA), un bis-PNA ou un ADN, par exemple un ADN simple brin (ADNss).

4. Procédé selon l'une quelconque des revendications 1 à 3, dans lequel la ou les plusieurs premières sondes et/ou la ou les plusieurs deuxièmes sondes comprennent un ou plusieurs oligonucléotides, par exemple un oligonucléotide constitué de 10 à 25 paires de bases et en particulier un oligonucléotide constitué de 10 à 15 paires de bases.

5. Procédé selon l'une quelconque des revendications 1 à 4, dans lequel la ou les plusieurs premières sondes et/ou la ou les plusieurs deuxièmes sondes comprennent une étiquette ou un marqueur détectable.

6. Procédé selon l'une quelconque des revendications 1 à 5, qui comprend un nanopore unique de moins de 10 nm de diamètre, par exemple de moins de 5 nm de diamètre et en particulier de 4 à 5 nm de diamètre.

7. Procédé selon l'une quelconque des revendications 1 à 6, dans lequel le nanopore comprend une membrane de nitrure de silicium (SiN).

8. Procédé selon l'une quelconque des revendications 1 à 7, dans lequel l'échantillon est un échantillon de plasma sanguin maternel qui comprend un acide nucléique foetal.

9. Procédé selon l'une quelconque des revendications 2 à 8, dans lequel l'anomalie comprend trois copies du chromosome 21 chez un sujet présentant le syndrome de Down par comparaison à deux copies chez un sujet qui en est exempt.

10. Utilisation d'un procédé selon l'une quelconque des revendications 1 à 9 pour déterminer si un individu présente une augmentation ou une réduction du nombre de copies d'un gène ou d'un chromosome.
